# EUROPEAN PATENT APPLICATION

(11) **EP 4 278 972 A1**
(43) Date of publication of application: **22.11.2023**
(21) Application number: 22739664.5
(22) Date of filing: 12.01.2022
(51) Int. Cl.: A61B 5/151, A61B 5/15

(54) **REPLACEMENT-TYPE BLOOD-COLLECTING DEVICE**

(30) Priority: 12.01.2021 KR 20210003831; 20.05.2021 KR 20210064536; 14.07.2021 KR 20210092247
(71) Applicant: Roahmed Co., Ltd., Changwon-Si, Gyeongsangnam-do 51573 (KR)
(72) Inventor: CHOI, Imcheol, Busan 46760 (KR)
(74) Representative: Gulde & Partner
(86) International application number: PCT/KR2022/000548
(87) International publication number: WO 2022/154469

(57) **Abstract**

Disclosed is a replaceable blood lancet device in which a needle unit including a needle carrier and a front slider is used as inserted in a tubular barrel, and the used needle unit or needle carrier is replaced after use. The replaceable blood lancet device minimizes waste of resources by reducing the parts to be discarded in reuse. In addition, the front slider inserted in the barrel is easily loaded and unloaded through the withdrawal grip portion provided in the front slider. Further, an action spring for elastically pushing the needle carrier in a frontward direction, and a rear end cap for supporting the actuation spring to be compressed to generate an elastic force are provided, so that the blood lancet device can perform blood collection based on the elastic force of the spring.

## Description

### [TECHNICAL FIELD]

The disclosure relates to a replaceable blood lancet device.

### [BACKGROUND ART]

A blood lancet device is used to draw a small amount of blood from a part of a body to perform test such as blood grouping and blood glucose measurement.

In general, the general blood lancet device includes a tubular barrel loaded with a needle carrier having a needle, and the loaded needle carrier is elastically fired so that the needle can puncture skin to draw a very small amount of blood. Once used, the needle carrier is replaced by disassembling its front end or removing the entire needle carrier to prevent infection due to reuse.

However, such a blood lancet device has a problem in that an economic efficiency is low because manufacturing costs are increased by a complex structure and many parts required to implement the attachment/detachment of the needle carrier and a needle unit including the needle carrier.

Further, there is a problem that the blood lancet device is inconvenient for elderly users to use because the parts are frequently broken or lost in the process of replacing the needle carrier and the needle unit and are difficult to assemble.

### [DISCLOSURE]

### [TECHNICAL PROBLEM]

An aspect of the disclosure is to provide a blood lancet device in which a needle unit is easily replaceable through an intuitive and simple detachable structure.

Another aspect of the disclosure is to provide a blood lancet device including a needle unit, in which shaking that occurs during blood collection due to the lock and release of the needle unit is minimized.

Still another aspect of the disclosure is to provide a blood lancet device having a function of adjusting the depth of the needle to be inserted into the epidermis.

Still another aspect of the disclosure is to provide a replaceable blood lancet device that can minimize pain caused by a needle while allowing the needle to be rapidly withdrawn.

Still another aspect of the disclosure is to provide a replaceable blood lancet device that can keep a needle hygienic until the needle is used.

Still another aspect of the disclosure is to provide a replaceable blood lancet device that can be effectively prevented from reuse.

### [TECHNICAL SOLUTION]

According to an embodiment of the disclosure, a replaceable blood lancet device includes a tubular barrel comprising a front opening; a needle unit inserted in the front opening of the barrel and slidably movable along an axial line direction; and an action spring elastically pushing the needle unit in the barrel outwards in a front end direction.

In addition, when the barrel includes a withdrawal opening opened laterally in a predetermined lengthwise section from the front opening to expose the needle unit or cut out in a predetermined section along a longitudinal direction to guide withdrawal of the needle unit, it is easy to withdraw the needle unit from the barrel through the withdrawal opening. Further, the withdrawal opening is useful even to insert the needle unit in the barrel.

In addition, when the barrel includes a rear opening, and further includes a rear end cap provided to open and close the rear opening and being in contact with a rear end of the actuation spring, the parts are inserted through the rear opening of the barrel and the rear opening is closed with the rear end cap, thereby facilitate the placement and assembly of the parts inside the blood lancet device.

In addition, when a needle depth adjuster is provided between the barrel and the rear end cap to adjust a position of the rear end cap in an axial line direction, the depth at which the needle for blood collection included in the needle unit is inserted in the epidermis is adjustable, thereby allowing the blood lancet device to be flexibly used according to the purposes of blood collection and users.

Further, when the needle depth adjuster includes a spiral guide portion formed in the barrel, and an engagement moving portion provided in the rear end cap and engaging with the spiral guide portion, the rear end cap is rotated from the barrel to minutely adjust the depth at which the needle is inserted in the epidermis.

In addition, when a stop bushing is additionally interposed between the barrel and the rear end cap, rotating with the rear end cap, and including a lock resistance portion formed between the stop bushing and the barrel and causing rotation resistance based on engagement therebetween, the depth at which the needle is inserted is kept constant by the lock resistance portion, and the stepwise depths are adjusted and maintained according to the grooves formed in the lock resistance portion.

In addition, when the needle unit includes: a tubular front slider inserted in the barrel and movable in the axial line direction, and including a front end partially exposed from an end portion of the barrel; and a needle carrier including a carrier body supporting a needle for blood collection to protrude in a front end direction and accommodated in the front slider movably in the axial line direction, a plurality of elastic transformation portions extending rearwards from the carrier body and elastically transformable inwards in a radial direction, and an interlocking holding protrusion protruding from the elastic transformation portion in a direction transverse to the axial line direction, held in a rear end of the front slider, and moving together with the front slider upon rearward movement, the needle unit is divided into the front slider and the needle carrier, and the interaction between the front slider and the needle carrier facilitates the standby and blood collection process of the blood lancet device.

Further, when the replaceable blood lancet device further includes a stopper protruding inwards in the radial direction of the front slider and kept held in the interlocking holding protrusion; and a separation preventing protrusion provided in the needle carrier, held in the stopper, and preventing the needle carrier from being separated in a rearward moving direction, it is possible to limit the movable range of the needle carrier and prevent the reuse of the needle carri er.

In addition, when a bumper spring is mounted in a front of the needle carrier and extends longer than a protruding length of the needle, the buffer spring can reduce pain by striking epidermis before the needle and prevent accidents caused by the used needle by preventing the exposure of the needle after blood collection.

In addition, when the replaceable blood lancet device further includes a standby lock groove provided in one of an inner wall of the barrel and an outer wall of the front slider; and a standby lock portion provided in the other one of the inner wall of the barrel and the outer wall of the front slider, locked to the standby lock groove, and allowing the front slider to be movable within a certain range, the front slider is prevented from separating by a certain extent or more in the front or rear end directions of the barrel, thereby preventing the front slider from incorrect operations during the standby and blood collection process.

In addition, when the front slider further includes a withdrawal grip portion to grip and withdraw one end of the front slider inserted in the barrel, a user can grip and withdraw the front slider to be forcibly separated as necessary. Further, the withdrawal grip portion may also be useful even when the front slider is loaded into the barrel.

Further, when the withdrawal grip portion is connected to the front slider through the withdrawal opening and exposed to the outside of the barrel or annularly surrounding the barrel, the grip region of the withdrawal grip portion is enlarged to facilitate the withdrawal of the front slider.

In addition, when the barrel includes a trigger portion provided therein, and a slope provided in one of the trigger portion and the elastic transformation portion provided in the needle carrier transforms the elastic transformation portion inwards upon retraction of the needle carrier, the needle carrier can be inserted into the front slider by the movement of the needle carrier or the barrel.

According to another embodiment of the disclosure, a needle unit of a replaceable blood lancet device including a tubular barrel includes: a tubular front slider inserted in the barrel and movable in the axial line direction, and including a front end partially exposed from an end portion of the barrel; and a needle carrier including a carrier body supporting a needle for blood collection to protrude in a front end direction and accommodated in the front slider movably in the axial line direction, a plurality of elastic transformation portions extending rearwards from the carrier body and elastically transformable inwards in a radial direction, and an interlocking holding protrusion protruding from the elastic transformation portion in a direction transverse to the axial line direction, held in a rear end of the front slider, and moving together with the front slider upon rearward movement.

Further, when the replaceable blood lancet device further includes a stopper protruding inwards in the radial direction of the front slider and kept held in the interlocking holding protrusion; and a separation preventing protrusion provided in the needle carrier, held in the stopper, and preventing the needle carrier from being separated in a rearward moving direction, it is possible to limit the movable range of the needle carrier and prevent the reuse of the needle carri er.

In addition, when there is a bumper spring mounted in a front of the needle carrier and extending longer than a protruding length of the needle, the bumper spring can reduce pain by striking the epidermis before the needle and prevent accidents caused by the used needle by preventing the exposure of the needle after blood collection.

In addition, when a standby lock portion is provided in the other one of the inner wall of the barrel and the outer wall of the front slider, locked to the standby lock groove, and allowing the front slider to be movable within a certain range, the front slider is prevented from separating by a certain extent or more in the front or rear end directions of the barrel, thereby preventing the front slider from incorrect operations during the standby and blood collection process.

In addition, when the front slider further includes a withdrawal grip portion to grip and withdraw one end of the front slider inserted in the barrel, a user can grip and withdraw the front slider to be forcibly separated as necessary. Further, the withdrawal grip portion may also be useful even when the front slider is loaded into the barrel.

Further, when the withdrawal grip portion is connected to the front slider through the withdrawal opening provided in the front end of the barrel and exposed to the outside of the barrel or annularly surrounding the barrel, the grip region of the withdrawal grip portion is enlarged to facilitate the withdrawal of the front slider.

In addition, when the front slider includes a projection coupling portion to prevent the needle carrier from being separated in a rearward direction, the needle carrier is prevented from being separated from the front slider in the rearward direction due to shaking, rotation, etc. of the blood lancet device.

### [ADVANTAGEOUS EFFECTS]

Ablood lancet device according to the disclosure has an intuitive and simple detachable structure through which a needle unit is easily replaceable.

Further, according to embodiments of the disclosure, there is provided a replaceable blood lancet device that has effects on minimizing shaking that occurs during a blood collection process by the lock and release of a needle unit, making it possible to adjust the depth of a needle inserted into epidermis, minimizing pain, maintaining the hygiene of the needle, and preventing the reuse of the needle unit.

### [DESCRIPTION OF DRAWINGS]

FIG. 1 is a perspective view of s replaceable blood lancet device according to an embodiment of the disclosure.
FIG. 2 is a longitudinal section view of a replaceable blood lancet device according to an embodiment of the disclosure.
FIG. 3 is an exploded perspective view of a replaceable blood lancet device according to an embodiment of the disclosure.
FIGS. 4 (a) to (c) are cross-sectional views illustrating an assembly process of a needle unit according to an embodiment of the disclosure.
FIGS. 5 (a) to (e) are cross-sectional views showing a use process in sequence.
FIG. 6 is a perspective view of a replaceable blood lancet device according to another embodiment of the disclosure.
FIG. 7 is an exploded perspective view of a replaceable blood lancet device according to another embodiment of the disclosure.
FIG. 8 is a longitudinal section view of a replaceable blood lancet device according to another embodiment of the disclosure.
FIGS. 9 (a) and (b) are views illustrating an assembly relationship between a barrel and a front slider in a replaceable blood lancet device according to another embodiment of the disclosure.
FIGS. 10 (a) to (e) are views sequentially illustrating a blood collection process of a replaceable blood lancet device according to another embodiment of the disclosure.
FIGS. 11 (a) and (b) are views illustrating the replacement of a needle carrier of a replaceable blood lancet device according to another embodiment of the disclosure.

### **DESCRIPTION OF REFERENCE NUMERALS * *

1: epidermis 2: needle hole
10, 10': blood lancet device 100, 100': barrel
110, 110': front opening 120, 120': withdrawal opening
130, 130': standby lock portion 140: trigger portion
141: slope 150: rear opening
151: spiral guide portion 152: lock resistance protrusion
160: indicator 200, 200': front slider
210, 210': standby holding protrusion 220: stopper
230: guide cutout portion 240, 240': withdrawal grip portion
241': slider guide 242': elastic cutout groove
250: projection coupling portion 300: needle carrier
310: carrier body 320: needle
330: elastic transformation portion 340: outward projection
341: interlocking holding protrusion 342: separation preventing protrusion
350: protective extending portion 360: interlocking supporting portion
400: actuation spring 500: rear end cap
520: rotation lock portion 530: spring supporter
540: bushing lock groove 550: sign
600: stop bushing 610: rotation lock groove
620: engagement moving portion 630: lock resistance portion
640: bushing protruding portion 700: bumper spring
800: actuation member

### [BEST MODE]

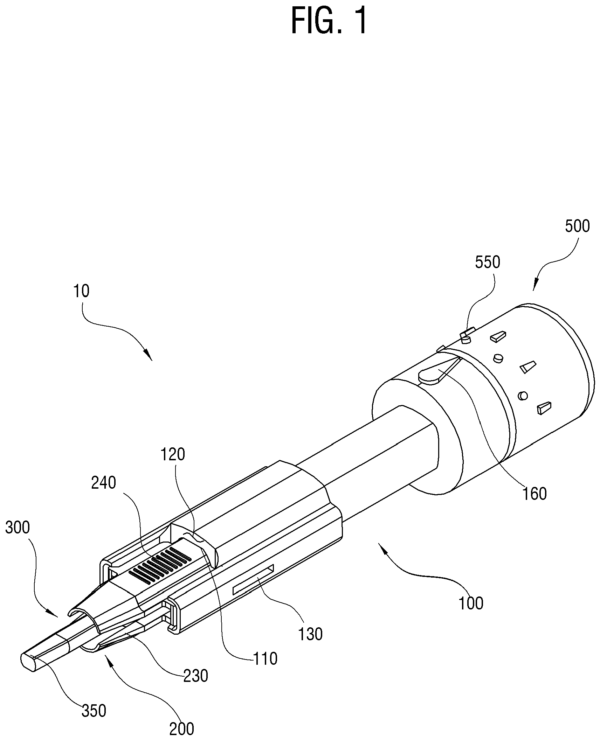

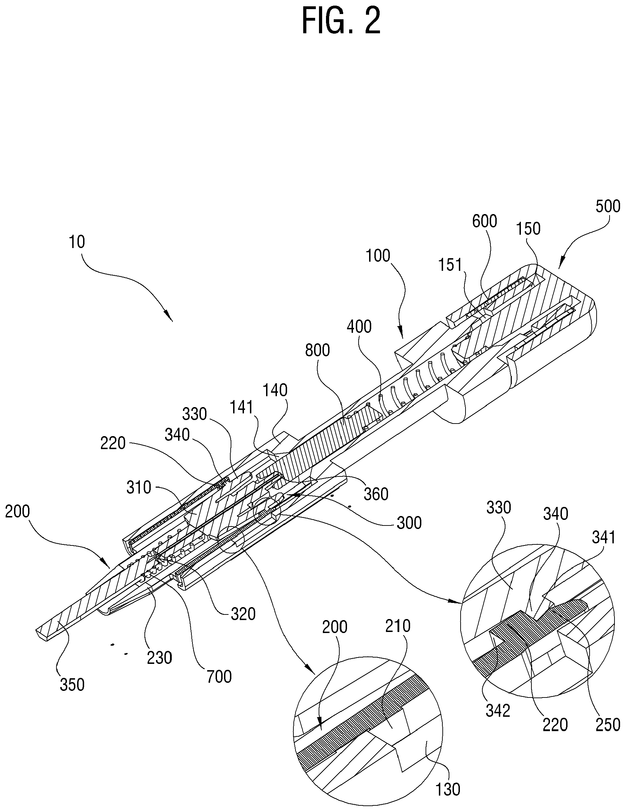

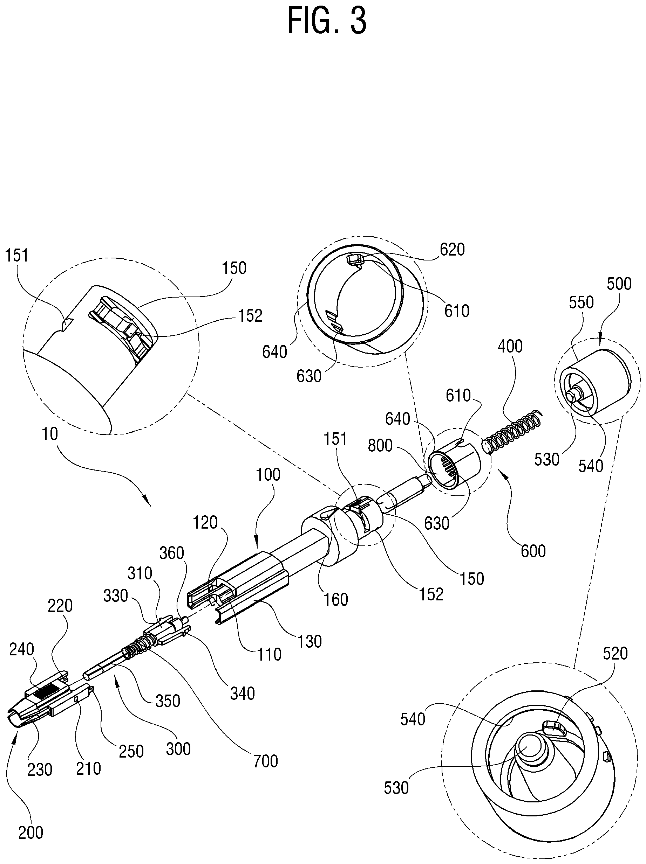

FIGS. 1 to 3 are a perspective view, an exploded perspective view and a longitudinal section view of a replaceable blood lancet device according to an embodiment of the disclosure. Detailed configurations of a replaceable blood lancet device according to an embodiment of the disclosure will be described with reference to FIGS. 1 to 3.

A replaceable blood lancet device 10 according to the disclosure refers to a device in which a needle unit including a front slider 200 and a needle carrier 300 is inserted in a tubular barrel 100, and the needle carrier 300 inserted in the barrel 100 is elastically fired by an actuation spring 400 interposed between the barrel 100 and the needle carrier 300, thereby collecting a small amount of blood from a small needle hole 2 formed in epidermis 1.

The barrel 100 has a front opening 110 that is open at a front end. Around the front opening 110 is formed a withdrawal opening 120, which is formed by cutting out a portion of the barrel 100. On one side of the front end of the barrel 100 is formed a standby lock portion 130, in which the needle unit is inserted and held. Inside the barrel 100 is formed a trigger portion 140. The rear end of the barrel 100 is also open like the front end thereof and has a rear opening 150, and an indicator 160 formed on the outer surface of the barrel 100.

The withdrawal opening 120 is cut out from the front end over a predetermined lengthwise section along an axial line direction and a predetermined widthwise section along a circumferential direction. The width and length of the withdrawal opening 120 are large enough to allow a finger of a user to come into contact with a surface of the front slider 200 included in the needle unit. There may be at least one withdrawal opening 120, and a pair of withdrawal openings 120 may be disposed facing each other along the circumferential direction of the front opening 110. Further, the withdrawal openings 120 may be arranged to guide the withdrawal of the front slider 200 included in the needle unit along the longitudinal direction of the cut section.

The standby lock portion 130 is shaped like a groove that limits a movable range of the front slider 200 of the needle unit so that the front slider 200 inserted in the interior of the barrel 100 cannot move beyond the front opening 110 or the interior of the barrel. Due to the standby lock portion 130, only the free end of the front slider 200 inserted into the barrel 100 is exposed, and the remaining portion is not exposed from the interior of the barrel 100. Further, even when the front slider 200 is moved to the rear end of the barrel 100, it may be ensured that one side of the front slider 200 is exposed to the front opening 110 or the withdrawal opening 120.

The trigger portion 140 refers to a portion that protrudes from the interior of the barrel 100, so that the one end of the needle carrier 300 touches the trigger portion 140 to limit the rearward movement of the needle carrier 300 or to cause one end of the needle carrier 300 to be affected when the needle carrier 300 is moved rearwards. A slope 141 is formed on the surface of the trigger portion 140 so that the needle carrier 300 can be gradually affected while moving along the longitudinal direction of the barrel 100.

The rear opening 150 is an opening formed at the rear end of the barrel 100. The rear opening 150 may facilitate the assembly of the blood lancet device 10 by allowing parts such as the actuation spring 400 except the needle unit inserted through the front opening 110 of the barrel 100 to be inserted therethrough and then being closed with a rear end cap 500 (to be described later). On the other hand, the rear end of the barrel 100 may be blocked not to form the rear opening 150 for some purposes, and in this case, the actuation spring 400 may be supported on the rear end of the barrel 100. The rear opening 150 includes a spiral guide portion 151 into which the rear end cap 500 (to be described later) is inserted, and a lock resistance protrusion 152 which limits a rotation angle of the rear end cap 500.

As described above, the needle unit includes the front slider 200 and the needle carrier 300.

The front slider 200 has a tubular shape along the axial line direction of the barrel 100. The front slider 200 is received through the front opening 110 of the barrel 100 and is partially exposed to the outside. A front end region of the front slider 200 may be shaped having an outer diameter that gradually increases from the front end toward the rear end along the length direction. The front slider 200 includes a standby holding protrusion 210 formed extending outwardly, a stopper 220 formed at the rear end of the front slider 200, a guide cutout portion 230 formed by partially cutting out a front end portion of the front slider 200, a withdrawal grip portion 240 formed on the outer surface or outer side of the front slider 200, and a projection coupling portion 250 formed at the rear end of the front slider 200.

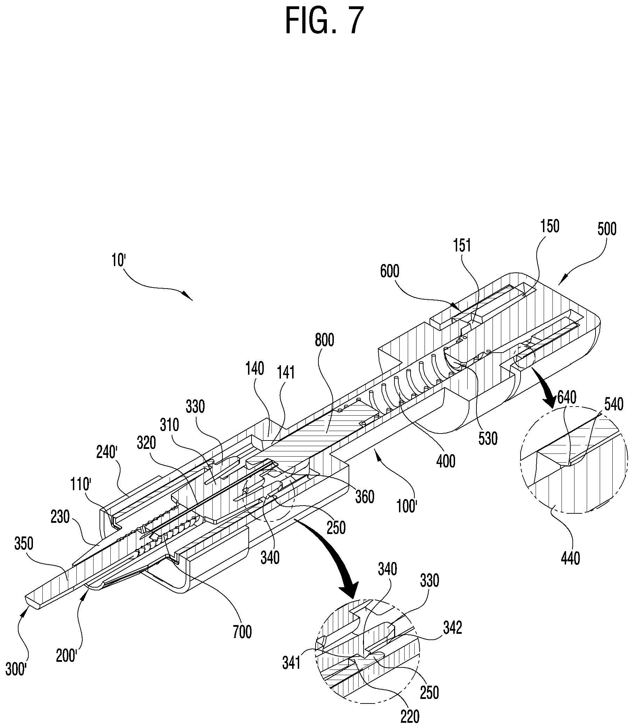

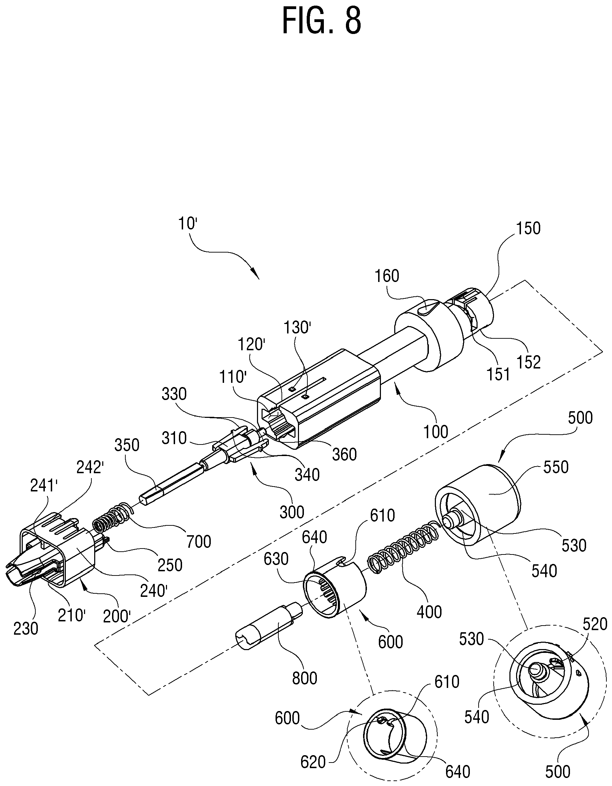

The standby holding protrusion 210 is formed to protrude from the outer side of the front slider 200 toward the standby lock portion 130 of the barrel 100. Thus, the movable range of the front slider 200 is limited so that the front slider 200 can move only within the section of a lock groove formed in the standby lock portion 130, and the front slider 200 is prevented from being separated from the barrel 100 as the standby holding protrusion 210 is locked to one end of the standby lock portion 130 in the case of simple movement to which a user's force is not applied. This coupling relationship between the protrusion and the groove can also be formed in reverse, like a standby lock portion 130' (see FIG. 8) and a standby holding protrusion 210' (see FIG. 8) formed in a front slider 200' (see FIG. 7) and a barrel 100' (see FIG. 7) according to another embodiment. Further, the shape of the standby lock portion 130 may be shaped like a protrusion to be arranged at a position spaced frontwardly or rearwardly apart from the standby lock portion 130, and the lock may be implemented as a standby holding protrusion (not shown) and a separation preventing protrusion (not shown) having protrusion shapes other than the groove shape, so that the standby holding protrusion 210 can be locked within a predetermined range.

The stopper 220 is formed at the end of a rear opening region of the front slider 200 and protrudes in the direction of the central axis of the front slider 200. The stopper 220 is interlocked with the frontward movement of the needle carrier 300 when the needle carrier 300 is located in a rear outer region of the front slider 200, thereby preventing the rear end portion of the needle carrier 300 from entering the interior of the front slider 200. In addition, the stopper 220 is interlocked with the rearward movement of the needle carrier 300 when the needle carrier 300 is in the front slider 200, thereby preventing the needle carrier 300 from separating in the direction of the rear end of the front slider 200.

The guide cutout portion 230 is formed in a front end region of the front slider 200 to have cut surfaces facing each other along a longitudinal direction, thereby preparing an opening region in a front end portion. The guide cutout portion 230 allows the front end portion of the front slider 200 to be elastically transformed. Therefore, the needle carrier 300 may be inserted and mounted even through the opening region at the front end of the front slider 200, and incorrect operations due to interference between the barrel 100 and the needle carrier 300 are prevented during the movement of the front slider 200 to thereby facilitate a sliding movement of the front slider 200. Further, the presence of the guide cutout portion 230 is also advantageous to solve molding technical problems related to the overall shape of the front slider 200 and the formation of the stopper 220.

The withdrawal grip portion 240 is formed on the outer side or surface of the front slider 200 so that a user can easily grip the front slider 200 to insert the front slider 200 into the front opening 110 of the barrel 100, or withdraw the front slider 200 inserted in the front opening 110. The withdrawal grip portion 240 may correspond to the outer surface of the front slider 200 exposed through the front opening 110 and the withdrawal opening 120 of the barrel 100, and may be formed protruding outwardly from the outer surface of the front slider 200 exposed through the front opening 110 and the withdrawal opening 120, or having fine protrusions formed from the outer surface of the front slider 200 to facilitate the gripping.

The projection coupling portion 250 refers to a portion that is locked to one end of the needle carrier 300 so that the needle carrier 300 cannot separate in the rearward direction of the front slider 200 when the front slider 200 and the needle carrier 300 are initially coupled to maintain the assembled state of the needle unit.

The needle carrier 300 is coupled to the rear end of the front slider 200 movably along the axial line direction of the front slider 200. The needle carrier 300 includes a carrier body 310 formed to accommodate a needle 320 for blood collection therein, and at least one elastic transformation portion 330 provided in an extension portion extended rearwards in the axial line direction at the rear end of the carrier body 310. Here, there may be a pair or plurality of elastic transformation portions 330 symmetrical to an axial line.

The elastic transformation portion 330 is elastically transformable in a radial direction of the needle carrier 300 due to its shape. The elastic transformation portion 330 is provided with an outward projection 340 at the rear end thereof, and the outward projection 340 includes a front side protrusion functioning as an interlocking holding protrusion 341 that is blocked by the rear end of the front slider 200 or the stopper 220. When the front slider 200 is moved rearwards by the interlocking between the interlocking holding protrusion 341 and the stopper 220, the needle carrier 300 is also moved rearwards. On the rearward side of the outward projection 340, which is opposite to the interlocking holding protrusion 341 formed in the outward projection 340, a separation preventing protrusion 342 is formed. When the needle carrier 300 is being inserted in the front slider 200, the separation preventing protrusion 342 is blocked by the stopper 220 so that the needle carrier 300 cannot move back and separate from the rear end of the front slider 200, thereby preventing the needle carrier 300 from rearward separation. Here, the interlocking holding protrusion 341 and the separation preventing protrusion 342 may be respectively formed in the elastic transformation portions 330 separated from each other.

At the front end of the needle carrier 300, a protective extending portion 350 is formed long. The protective extending portion 350 may be formed integrally with a carrier body 310 upon the formation of the carrier body 310 while surrounding the needle 320. The protective extending portion 350 serves to hygienically protect the needle 320 until using the needle 320 and to prevent deformation of the needle 320. A manipulation tube (not shown) may be disposed to externally cover a connection portion between the protective extending portion 350 and the carrier body 310. The manipulation tube (not shown) may resist external forces acting from the protective extending portion 350 in the radial direction, thereby protecting the protective extending portion 350 not to be easily removed until the manipulation tube (not shown) is removed.

At the rear end of the needle carrier 300, there is an interlocking supporting portion 360 that supports one end of the actuation spring 400 or one end of the actuation member 800 (to be described later). When the interlocking supporting portion 360 is directly connected to the actuation spring 400, the length of the actuation spring 400 that presses the needle carrier 300 is increased to reduce the durability of the actuation spring 400 and cause a problem that the actuation spring 400 is easily damaged due to repeated elastic transformation. Therefore, the actuation member 800 may be interposed between the interlocking supporting portion 360 and the actuation spring 400..

The actuation member 800 may indirectly transmit the elastic force from the actuation spring 400 to the interlocking supporting portion 360 of the needle carrier 300. Because the length of the actuation spring 400 is shortened due to the actuation member 800, an inexpensive actuation spring 400 with an appropriate elastic force may be used, and the actuation spring 400 may be improved in stability and durability.

The actuation spring 400 provides a driving force to elastically fire the needle carrier 300 included in the needle unit in the frontward direction. Thus, the needle unit, i.e., the needle carrier 300 and the front slider 200, is subjected to an elastic resistance force at the rearward movement. The actuation spring 400 may be supported by the rear end of the barrel 100 when the rear end of the barrel 100 is closed, but the actuation spring 400 may be supported by the rear end cap 500 that covers the rear end of the barrel 100 when the rear end of the barrel 100 is open through the rear opening 150.

The rear end cap 500 is coupled to the rear end of the barrel 100 through the rear opening 150 of the barrel 100, and a stop bushing 600 is interposed between the rear end cap 500 and the rear opening 150.

The rear end cap 500 covers the rear opening 150 of the barrel 100 to prevent parts such as the actuation spring 400 inserted in the barrel 100 or the needle unit from separating out of the rear opening 150, and adjusts the insertion depth of the needle 320 for blood collection. When the adjustment of the insertion depth of the needle 320 is not required, the blood lancet device 10 may also be configured with the barrel 100 having the rear opening 150 closed excluding the rear end cap 500 and the stop bushing 600.

When the stop bushing 600 is not interposed between the rear end cap 500 and the barrel 100, an engagement moving portion (not shown) may be provided in the rear end cap 500 and allows the rear end cap 500 to directly engage with the spiral guide portion 151 formed around the rear opening 150 of the barrel 100. When the rear end cap 500 and the rear opening 150 are directly coupled, the rear end cap 500 may rotate in a certain range by a groove area formed by the spiral guide portion 151, and such a spiral shape causes the rear end cap 500 to move up and down. On the other hand, the engagement moving portion (not shown) and the spiral guide portion 151, which are respectively provided in the rear end cap 500 and the barrel 100, may also be formed in reverse, i.e., implemented as an engagement moving portion (not shown) formed in the barrel 100 and a spiral guide portion (not shown) formed in the rear end cap 500. Further, such a rotatable coupling structure may also be implemented in a screw-like form with the engagement moving portion (not shown) formed on one of the rear end cap 500 or the rear opening 150 and the spiral guide portion (not shown) formed on the other. However, in this case, the rear end cap 500 is rotatable but not fixed at a certain point, and therefore the set depth of the needle may be often varied depending on an external force. To prevent this case, the stop bushing 600 may be interposed between the rear end cap 500 and the rear opening 150.

When the stop bushing 600 is interposed between the rear end cap 500 and the rear opening 150, the rear end cap 500 includes a rotation lock portion 520 to be locked to one end of the stop bushing 600 to interlock with the rotation of the rear end cap 500, a spring supporter 530, a bushing lock groove 540, and a sign 550.

The rotation lock portion 520 is coupled to the stop bushing 600 such that the rotational movement of the rear end cap 500 is interlocked with the stop bushing 600.

The spring supporter 530 refers to a portion that protrudes from the inner surface of the rear end cap 500 and supports the actuation spring 400.

The bushing lock groove 540 refers to a groove formed along an inner circumferential direction of the rear end cap 500 such that one end of the stop bushing 600 is inserted in the bushing lock groove 540 to assist the engagement between the rear end cap 500 and the stop bushing 600.

The sign 550 refers to a symbol or character mark disposed on the outer surface of the rear end cap 500. A user can intuitively determine the depth of the needle adjusted by the rear end cap 500 rotated to align the sign 550 with the indicator 160 formed on the outer surface of the barrel 100.

The stop bushing 600 refers to a cylindrical member formed along the circumferential direction of the rear opening 150, and is interposed between the rear end cap 500 and the rear opening 150 of the barrel 100 to allow the rear end cap 500 to stop at a specified rotation angle. The stop bushing 600 includes a rotation lock groove 610 to be engaged with the rotation lock portion 520 formed in the rear end cap 500, an engagement moving portion 620 to be engaged with the spiral guide portion 151 formed in the rear opening 150, a lock resistance portion 630 to be engaged with the lock resistance protrusion 152 formed in the rear opening 150, and a bushing protruding portion 640.

The rotation lock groove 610 refers to a groove that accommodates the rotation lock portion 520 formed in the rear end cap 500. When the rear end cap 500 is rotated, the stop bushing 600 also rotates interlocking with the rotation of the rear end cap 500.

The engagement moving portion 620 is inserted into the spiral guide portion 151 formed in the rear opening 150 to allow the stop bushing 600 to rotate within a certain range by the groove area formed by the spiral guide portion 151. Because the rotation of the rear end cap 500 is interlocked with the rotation lock portion 520 and the rotation lock groove 610 of the stop bushing 600, the stop bushing 600 is rotated by the rotation of the rear end cap 500, and the rear end cap 500 moves up and down while rotating along the shape of the spiral guide portion 151. The engagement moving portion 620 and the spiral guide portion 151, which are respectively provided in the stop bushing 600 and the barrel 100, may also be implemented in reserve as the engagement moving portion (not shown) formed on the barrel 100 and the spiral guide portion (not shown) formed on the stop bushing 600. The engagement moving portion 620 and the spiral guide portion 151 may also be implemented as a screw-like form in which both the engagement moving portion 620 and the spiral guide portion 151 have the same protruding or recessed shape.

The lock resistance portion 630 refers to a groove formed along a radial direction of the inner surface of the stop bushing 600. To this end, a plurality of grooves may be formed at regular intervals. When the rear end cap 500 is rotated, the stop bushing 600 is rotated together by the engagement relationship between the rotation lock groove 610 and the rotation lock portion 520, and each groove included in the lock resistance portion 630 is locked to the lock resistance protrusion 152 so that the rear end cap 500 can be stopped at regular angles. In this case, the lock resistance protrusion 152 may be formed to have elasticity as its periphery is partially cut.

The bushing protruding portion 640 is inserted in the bushing lock groove 540 of the rear end cap 500, thereby allowing the rear end cap 500 and the stop bushing 600 to be locked and coupled to each other.

Meanwhile, the front end of the needle carrier 300 is mounted with the bumper spring 700 surrounding the needle 320 and the protective extending portion 350. The bumper spring 700 extends further in the frontward direction than the needle 320, and thus strikes and presses against a user's skin before the needle 320 when the needle carrier 300 is driven for the blood collection in the frontward direction of the barrel 100. When the bumper spring 700 touches the epidermis of the user, it strikes and disrupts tactile nerves around the blood collection point. As the bumper spring 700 is compressed, it compresses the periphery of the epidermis to be punctured by the needle 320, thereby blocking nociceptive nerves around the epidermis. While the tactile nerves are disturbed and the nociceptive nerves are blocked as above, the epidermis is punctured by the needle 320 to make a user feel less pain. The compressed bumper spring 700 returns to its original state by generating the resilience. Thus, the needle 320 is quickly pulled out of the epidermis, thereby reducing pain during such a pulling-out operation. The bumper spring 700 may be provided in the form of a compressive coil spring as shown, but in some cases may be provided in the form of an elastic material such as a leaf spring, and silicone.

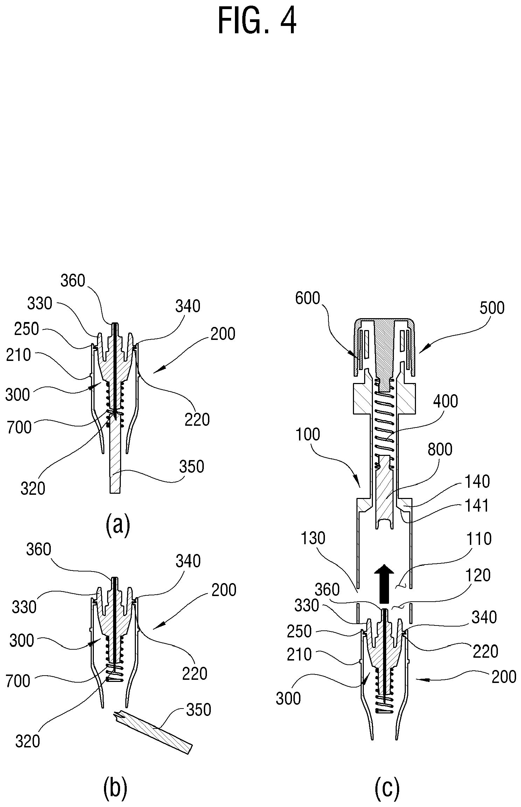

Referring to (a) through (c) of FIG. 4, the assembly process of the needle unit according to the disclosure will be described.

The bumper spring 700 is coupled to the front end of the needle carrier 300 as shown in (a) of FIG. 4, and inserted in the rear end of the front slider 200. In this case, the outward projection 340 formed on the needle carrier 300 is interposed between the stopper 220 and the projection coupling portion 250 at the rear end of the front slider 200, so that the needle carrier 300 can be coupled to the front slider 200.

The protective extending portion 350 may be removed from the front end of the needle carrier 300 assembled as shown in (b) of FIG. 4. The protective extending portion 350 may also be removed while the front slider 200 is inserted into the barrel 100.

The needle unit into which the front slider 200 and the needle carrier 300 are assembled is inserted in the front opening 110 of the barrel 100 by gripping the withdrawal grip portion 240 formed on the surface of the front slider 200 as shown in (c) of FIG. 4. In this time, a user can easily mount the needle unit to the barrel 100 through a space formed by the withdrawal opening 120 formed along the longitudinal direction of the barrel 100.

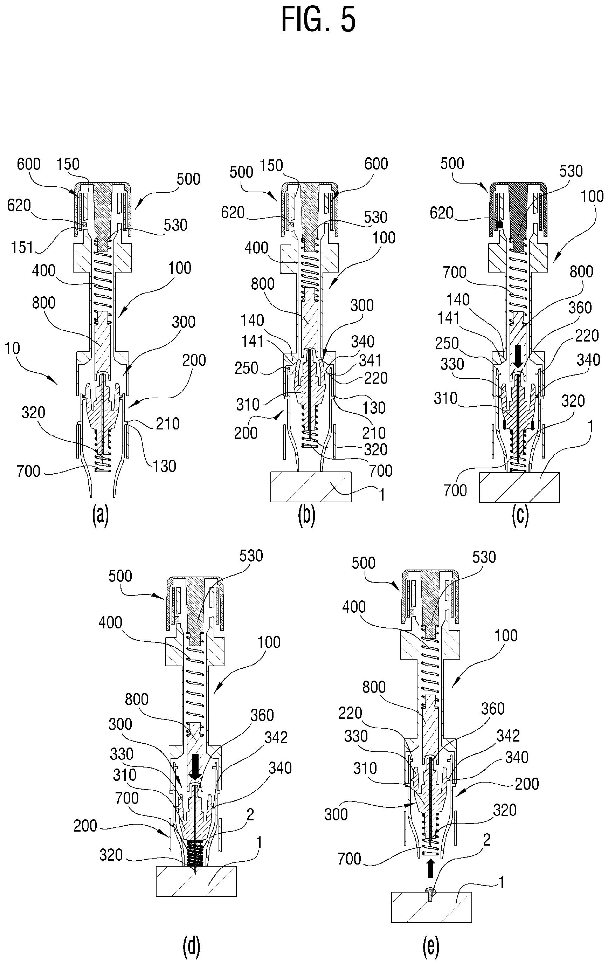

The process of using the blood lancet device 10 will be described with reference to (a) to (e) of FIG. 5.

(a) of FIG. 5 is a cross-sectional view illustrating a standby state of the blood lancet device 10 in which the needle unit is assembled through the steps (a) to (c) of FIG. 4. The needle 320 for blood collection is exposed at the front end of the needle carrier 300, and the frontward separation of the needle unit is prevented by the engagement between the standby holding protrusion 210 of the front slider 200 and the standby lock portion 130 of the barrel 100. Thus, the needle unit can maintain a standby position within the barrel 100.

In this state, when the front end of the blood lancet device 10, i.e., the front end of the front slider 200, is placed on the epidermis 1 of a person to be subjected to blood sampling and the barrel 100 is pressurized, the front slider 200 moves from the interior of the barrel 100 to the rear end.

(b) of FIG. 5 illustrates that the blood lancet device 10 moves close to the epidermis 1 of the blood sample and is in a retracted position in which the front slider 200 is moved rearwardly inside the barrel 100.

In this case, the stopper 220 of the front slider 200 and the outward projection 340 of the needle carrier 300 are interlocked, thereby causing the needle carrier 300 to move together in the rearward direction. When the needle carrier 300 moves in the rearward direction of the barrel 100, the elastic transformation portion 330 integrally formed with the needle carrier 300 comes into contact with the trigger portion 140 provided on the inner wall of the barrel 100, and the elastic transformation portion 330 is elastically transformed as being guided to the inner side of the barrel 100 by the slope 141 provided on the trigger portion 140. When this movement continues and the elastic transformation portion 330 is elastically transformed to a certain extent or more, a width between the opposite outward projections 340 becomes narrower than the inner width of the stopper 220 formed in the front slider 200, thereby releasing a blocking operation of the outward projection 340, and allowing the needle carrier 300 to enter the interior of the front slider 200 and to move in the front end direction. Meanwhile, the slope causing the elastic transformation may be provided in not the trigger portion 140 but the elastic transformation portion 300.

Meanwhile, as the needle carrier 300 is moved in the rearward direction by the front slider 200, the actuation member 800 connected to the interlocking supporting portion 360 is also moved in the rearward direction, the actuation spring 400 interposed between the spring supporter 530 and the actuation member 800 is compressed, and the elastic force of the compressed actuation spring 400 generates a force that propels the needle carrier 300 to move down and reach the epidermis 1.

(c) of FIG. 5 illustrates a moment when the needle carrier 300 is fired frontward by the compressed actuation spring 400 and the bumper spring 700 touches the epidermis. The needle carrier 300 receives the elastic force of the actuation spring 400 through the actuation member 800 and moves rectilinearly to the epidermis 1, and the bumper spring 700 provided in the front of the carrier body 310 strikes the epidermis 1. After the bumper spring 700 strikes the epidermis 1, the continued inertial motion of the needle carrier 300 causes the bumper spring 700 to be vibrated and compressed, and resilience is stored for the retraction of the needle carrier 300.

(d) of FIG. 5 illustrates that the needle 320 for blood collection penetrates into the body of a user and forms the needle hole 2. In this case, the needle 320 is fired with minimal transverse shaking from the central axis of the needle carrier 300 by the rectilinear motion of the needle carrier 300 and the actuation member 800 surrounding the interlocking supporting portion 360 of the needle carrier 300, thereby forming the needle hole 2 at an exactly desired point without shaking.

When the inertial force exerted by the needle carrier 300 causes the bumper spring 700 to be compressed to a certain extent or more, the needle 320 for blood collection is propelled rearward by the resilience of the bumper spring 700 and thus pulled out of the epidermis 1, thereby returning to the interior of the front slider 200.

(e) of FIG. 5 illustrates that the front slider 200 and the needle carrier 300 are returned after the blood collection process. Under the resilience of the compressed the bumper spring 700, the needle 320 for blood collection is quickly pulled out of the epidermis 1 and returns to the interior of the front slider 200, and the front slider 200 is freely positioned between the retraction position and the standby position because it is not under any force in either the frontward or rearward direction.

In this case, the used needle carrier 300 is located in the inner space of the front slider 200. When the needle carrier 300 is moved in the rearward direction of the front slider 200, the separation preventing protrusion 342 formed in the outward projection 340 is blocked by the stopper 220, so that the needle carrier 300 cannot be separated from the interior of the front slider 200 in the rearward direction and cannot be reused.

Thus, the used needle unit including the front slider 200 and the needle carrier 300 is separable from the barrel 100 by gripping it through the withdrawal opening 120 provided on a lateral side of the barrel 100, and the separated needle unit is discarded. Then, a new needle unit is inserted and used.

The foregoing blood lancet device 10 according to an embodiment of the disclosure is provided with the needle unit including the front slider 200 and the needle carrier 300, which can be inserted or withdrawn through the front opening 110 of the barrel 100.

Further, the needle unit can be easily loaded to and unloaded from the barrel 100 through the withdrawal opening 120 formed in the barrel 100 and the withdrawal grip portion 240 formed in the front slider 200.

Further, the rear end cap 500 allows the insertion depth to be adjusted depending on the skin thickness of a blood collection subject or the purpose of the blood collection.

Further, according to an embodiment of the disclosure, a compact structure and good operability are provided.

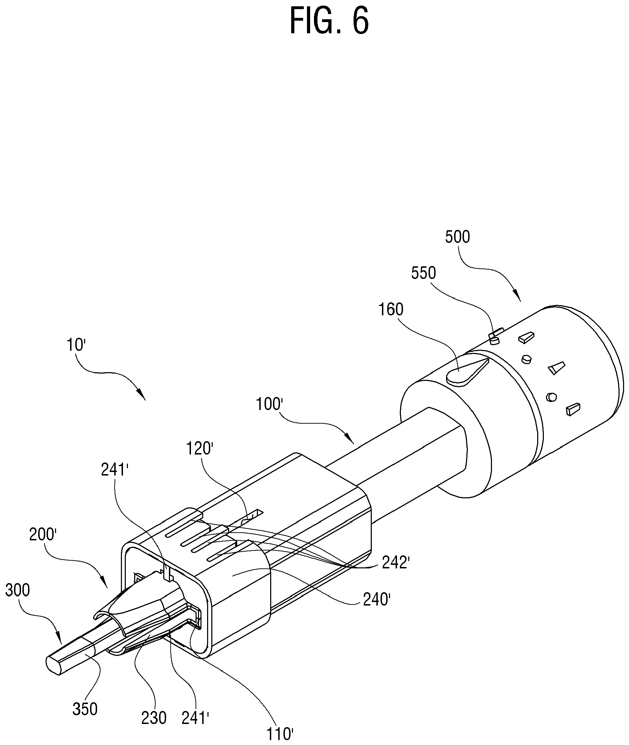

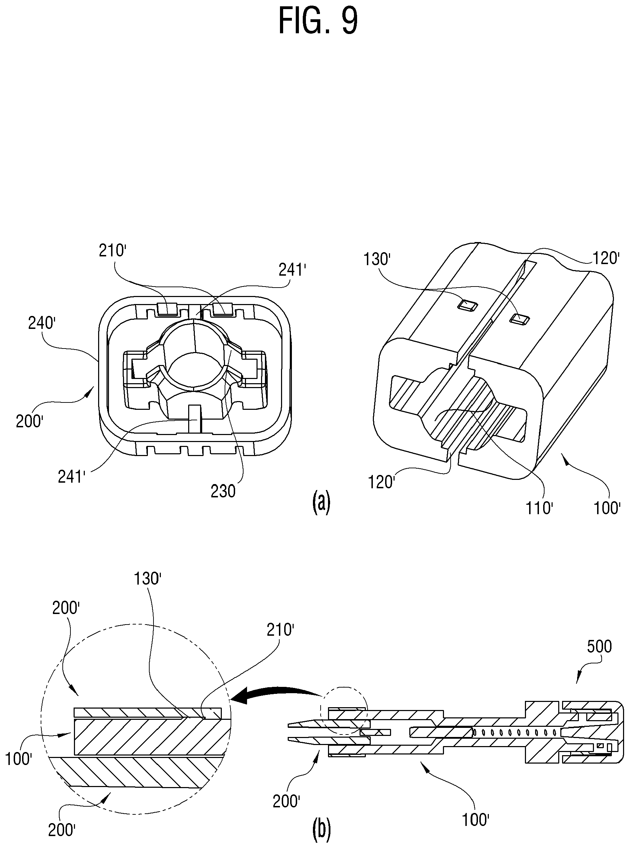

FIGS. 6 to 9 (a) and (b) are perspective, exploded, and longitudinal section views of a blood lancet device according to another embodiment of the disclosure, illustrating an assembly relationship between a barrel 100' and a front slider 200' of a blood lancet device 10'.

Referring to FIGS. 6 to 9 (a) and (b), the configuration of the blood lancet device according to another embodiment of the disclosure will be described. The replaceable blood lancet device 10' according to another embodiment of the disclosure includes a barrel 100', a front slider 200', a needle carrier 300, an actuation spring 400 and a rear end cap 500 that supports the actuation spring 400 and covers the rear end of the barrel 100'.

The barrel 100' is formed of a tubular body, and includes a front opening 110', a withdrawal opening 120', a standby lock portion 130', a trigger portion 140, a rear opening 150, and a sign 160.

The front slider 200' and the needle carrier 300 are accommodated in the barrel 100' through the front opening 110' opened at the front end of the barrel 100'. The withdrawal opening 120' is cut open in a predetermined section along a lengthwise direction from the front opening 110' to serve as a guide for accommodating or withdrawing the front slider 200' in or from the barrel 100'. The standby lock portion 130' is formed protruding from the surface of the barrel 100' and performs a holding operation to prevent the front slider 200' assembled in the barrel 100' from being separated in the front end direction.

On the inner wall of the barrel 100' is provided the trigger portion 140 having the slope 141 that gradually slopes inwardly toward the rear end at a position spaced rearwardly apart from the front opening 110'.

In the rear opening 150 opened at the rear end portion of the barrel 100', the rear end cap 500 is inserted. Around the rear opening 150 are formed the spiral guide portion 151 to which the rear end cap 500 is coupled, and the lock resistance protrusion 152 which causes the rear end cap 500 to stop at certain intervals along the circumferential direction of the rear opening 150.

The front slider 200' refers to a tubular member accommodated in the front end portion of the barrel 100', and has the front end portion being partially exposed from the front opening 110' of the barrel 100'. The front slider 200' has a withdrawal grip portion 240' formed annularly around the front opening 110' of the barrel 100', and includes a standby holding protrusion 210', a stopper 220, a guide cutout portion 230, and a projection coupling portion 250. Here, the withdrawal grip portion 240' is provided to be connected to the front slider 200' through the withdrawal opening 120', and exposed to the outside of the barrel 100'.

The withdrawal grip portion 240' allows a user to easily assemble or disassemble the front slider 200' from the barrel 100' by gripping the withdrawal grip portion 240'. The withdrawal grip portion 240' includes a slider guide 241' and an elastic cutout groove 242'. The slider guide 241' is interposed between the outer surface of the front slider 200' and the withdrawal grip portion 240' to support the withdrawal grip portion 240'. The slider guide 241' may help the front slider 200' to move straightly along a groove formed in the withdrawal opening 120' formed in the barrel 100'. The elastic cutout groove 242' refers to a cutout portion that allows a portion of the withdrawal gripping portion 240' to have elasticity. The elastic cutout groove 242' is formed on the periphery of the standby holding protrusion 210' so that the periphery of the standby holding protrusion 210' can be elastically transformed when the front slider 200' is assembled to or disassembled from the barrel 100'. Further, due to the elastic cutout groove 242', the standby holding protrusion 210' and the standby lock portion 130' do not easily wear out even though the standby holding protrusion 210' is repeatedly rubbed over a long period of time.

The standby holding protrusion 210' accommodates the standby lock portion 130' formed on the outer surface of the barrel 100' to prevent the front slider 200' from being separated from the barrel 100' in the front end direction. Because the standby lock portion 130' and the standby holding protrusion 210' are formed on the opposite wall surfaces of the barrel 100' and the front slider 200', the standby lock portion may be formed on one of the barrel 100' and the front slider 200', and the standby holding protrusion may be formed on the other of the barrel 100' and the front slider 200', thereby being locked to each other.

The stopper 220 is formed at the end of a rear opening region of the front slider 200 and protrudes in the direction of the central axis of the front slider 200. The stopper 220 is interlocked with the frontward movement of the needle carrier 300 when the needle carrier 300 is located in a rear outer region of the front slider 200, thereby preventing the rear end portion of the needle carrier 300 from entering the interior of the front slider 200. In addition, the stopper 220 is interlocked with the rearward movement of the needle carrier 300 when the needle carrier 300 is in the front slider 200, thereby preventing the needle carrier 300 from separating in the direction of the rear end of the front slider 200.

The guide cutout portion 230 is formed in a front end region of the front slider 200 to have cut surfaces facing each other along a longitudinal direction, thereby preparing an opening region in a front end portion. The guide cutout portion 230 allows the front end portion of the front slider 200 to be elastically transformed. Therefore, the needle carrier 300 may be inserted and mounted even through the opening region at the front end of the front slider 200, and incorrect operations due to interference between the barrel 100 and the needle carrier 300 are prevented during the movement of the front slider 200 to thereby facilitate a sliding movement of the front slider 200. Further, the presence of the guide cutout portion 230 is also advantageous to solve molding technical problems related to the overall shape of the front slider 200 and the formation of the stopper 220.

The guide cutout portion 230 refers to a portion formed by cutting out opposite sides of the front slider 200' along the lengthwise direction. The guide cutout portion 230 is advantageously utilized to solve molding technical problems related to the shape of the front slider 200', and allows the width of the front slider 200' to be flexibly varied due to the cutout region thereof so that parts driven inside the front slider 200' can be smoothly moved.

The projection coupling portion 250 is formed at the rear end of the front slider 200' to insert and couple the needle carrier 300 in and with the front slider 200', thereby preventing the rearward separation so that the coupling between the front slider 200' and the needle carrier 300 cannot be released until firing after the front slider 200' and the needle carrier 300 are assembled through the front opening 110' of the barrel 100'.

The needle carrier 300 includes a carrier body 310, a needle 320 for blood collection, an elastic transformation portion 330, an outward projection 340, and a protective extending portion 350. The carrier body 310 is mounted with the blood collection needle 320 at the front end thereof, and includes a pair of elastic transformation portions 330 provided at the rear end thereof and extending rearwards in axial symmetry. These elastic transformation portions 330 are elastically transformable in a radial direction.

The elastic transformation proportion 330 is provided with an outward projection 340 at the rear end thereof. The outward projection 340 is interposed between the stopper 220 and the projection coupling portion 250 of the front slider 200' and keeps coupling between the needle carrier 300 and the front slider 200'. The interlocking holding protrusion 341 formed on one side of the outward projection 340 is blocked by the stopper 220 when the front slider 200' moves rearwards, so that the needle carrier 300 can also move rearwards together with the front slider 200'.

At the front end of the needle carrier 300, the protective extending portion 350 is extended long in the direction of the front end. The protective extending portion 350 is formed integrally with the carrier body 310 upon the molding of the carrier body 310 while surrounding the needle 320. The protective extending portion 350 serves to hygienically protect the blood collection needle 320. The protective extending portion 350 may be protected from the outside by a manipulation tube (not shown) that covers the exterior thereof.

At the rear end of the needle carrier 300, an interlocking supporting portion 360 is formed. The interlocking supporting portion 360 accommodates a portion of the actuation member 800 (to be described later).

The actuation spring 400 is interposed between the other end of the actuation member 800 being in contact with the interlocking supporting portion 360 and the rear end cap 500 (to be described later), compressed by the movement of the needle carrier 300, and then transmit the compressed elastic force back to the needle carrier 300, thereby elastically pushing the needle carrier 300 in the frontward direction.

The rear end cap 500 is coupled to the rear opening 150 of the barrel 100' and shields the opening of the rear opening 150. The rear end cap 500 is rotated along the radial direction of the rear opening 150, and provided as a set with the stop bushing 600 (to be described later), so that the rear end cap 500 can stop at certain points within a rotation section. The rear end cap 500 includes a rotation lock portion 520, a spring supporter 530, a bushing lock groove 540, and a sign 550.

The rotation lock portion 520 is coupled to the stop bushing 600 such that the rotational movement of the rear end cap 500 is interlocked with the stop bushing 600.

The spring supporter 530 refers to a portion that protrudes from the inner surface of the rear end cap 500 and supports the actuation spring 400.

The bushing lock groove 540 refers to a groove formed along an inner circumferential direction of the rear end cap 500 such that one end of the stop bushing 600 is inserted in the bushing lock groove 540 to assist the engagement between the rear end cap 500 and the stop bushing 600.

The sign 550 refers to a symbol or character mark disposed on the outer surface of the rear end cap 500. A user can intuitively determine the depth of the needle adjusted by the rear end cap 500 rotated to align the sign 550 with the indicator 160 formed on the outer surface of the barrel 100.

The stop bushing 600 refers to a cylindrical member formed along the circumferential direction of the rear opening 150, and is interposed between the rear end cap 500 and the rear opening 150 of the barrel 100 to allow the rear end cap 500 to stop at a specified rotation angle. The stop bushing 600 includes a rotation lock groove 610 to be engaged with the rotation lock portion 520 formed in the rear end cap 500, an engagement moving portion 620 to be engaged with the spiral guide portion 151 formed in the rear opening 150, a lock resistance portion 630 to be engaged with the lock resistance protrusion 152 formed in the rear opening 150, and a bushing protruding portion 640.

The rotation lock groove 610 refers to a groove that accommodates the rotation lock portion 520 formed in the rear end cap 500. When the rear end cap 500 is rotated, the stop bushing 600 also rotates interlocking with the rotation of the rear end cap 500.

The engagement moving portion 620 is inserted into the spiral guide portion 151 formed in the rear opening 150 to allow the stop bushing 600 to rotate within a certain range by the groove area formed by the spiral guide portion 151. The engagement between the rotation lock groove 610 and the rotation lock portion 520 allows the stop bushing 600 to rotate together with the rotation of the rear end cap 500.

The lock resistance portion 630 refers to a groove formed along a radial direction of the inner surface of the stop bushing 600. To this end, a plurality of grooves may be formed at regular intervals. When the rear end cap 500 is rotated, the stop bushing 600 is rotated together by the engagement relationship between the rotation lock groove 610 and the rotation lock portion 520, and each groove included in the lock resistance portion 630 is locked to the lock resistance protrusion 152 so that the rear end cap 500 can be stopped at regular angles. In this case, the lock resistance protrusion 152 may be formed to have elasticity as its periphery is partially cut.

The bushing protruding portion 640 is inserted in the bushing lock groove 540 of the rear end cap 500, thereby allowing the rear end cap 500 and the stop bushing 600 to be locked and coupled to each other.

Referring to FIGS. 6 to 9 (a) and (b), the assembly process of the replaceable blood lancet device 10' according to the disclosure will be described.

The bumper spring 700 is coupled to the front end of the needle carrier 300 and the needle carrier 300 is inserted in the rear end of the front slider 200'. In this case, the bumper spring 700 extends longer in the front end direction than the needle 320 for blood collection, so that the bumper spring 700 can touch and press the skin of a user before the needle 320 for the blood collection when the needle carrier 300 is fired, thereby disturbing tactile sensation around a contact area first and then blocking nociception, thereby reducing pain of a user. The bumper spring 700 may be provided in the form of a coil spring as shown, but in some cases may be provided in the form of an elastic material such as a leaf spring, and silicone.

At this time, the outward projection 340 formed on the needle carrier 300 is interposed between the rear end of the front slider 200' and the projection coupling portion 250, so that the needle carrier 300 can be in a state of being coupled to the front slider 200'.

The protective extending portion 350 is then removed from the front end of the assembled needle carrier 300. The removal of the protective extending portion 350 may also be performed even while the front slider 200' is being inserted in the barrel 100'.

The front slider 200' with the needle carrier 300 inserted therein is inserted in the barrel 100' through the front opening 110'. In this case, the slider guide 241' is inserted in the withdrawal opening 120' formed along the longitudinal direction of the barrel 100' and assembled to be guided by each other.

In this case, the elastic cutout groove 242' formed in the withdrawal grip portion 240' is formed around the standby holding protrusion 210' so that the standby holding protrusion 210', which is affected by the standby lock portion 130' formed in the barrel 100', can be elastically transformed to facilitate the assembly of the front slider 200'.

Meanwhile, the rear end cap 500 is coupled to the rear end of the barrel 100'. Before the rear end cap 500 is coupled to the rear end of the barrel 100', the stop bushing 600 is first fitted to the rear end portion of the barrel 100'. When the stop bushing 600 is assembled, the lock resistance portion 630 formed in the interior of the stop bushing 600 comes into contact with the lock resistance protrusion 152 formed around the rear opening 150 of the barrel 100'. Because the stop bushing 600 is forcibly fitted to the rear end portion of the barrel 100', the stop bushing 600 is made of an easily contractable and expandable material so as to contract and return back to its original size.

Then, prior to coupling the rear end cap 500 to the stop bushing 600 and the rear opening 150, the actuation spring 400 is connected to the spring supporter 530. At the other end of the connected actuation spring 400, the actuation member 800 is coupled to form a connection body among the rear end cap 500, the actuation spring 400, and the actuation member 800. The rear end cap 500 forming this connection body is coupled to the stop bushing 600 and the spiral guide portion 151 formed in the rear opening 150.

Referring back to the assembly relationship between the barrel 100' and the front slider 200' coupling with the needle carrier 300' and the actuation member 800, when the front slider 200' is mounted through the front opening 110' of the barrel 100', the interlocking supporting portion 360 provided at the rear end of the needle carrier 300 touches and pushes the actuation member 800 in the rear end direction of the barrel 100', and the actuation spring 400 is compressed by a certain extent while pressing the needle carrier 300 inserted in the barrel 100'.

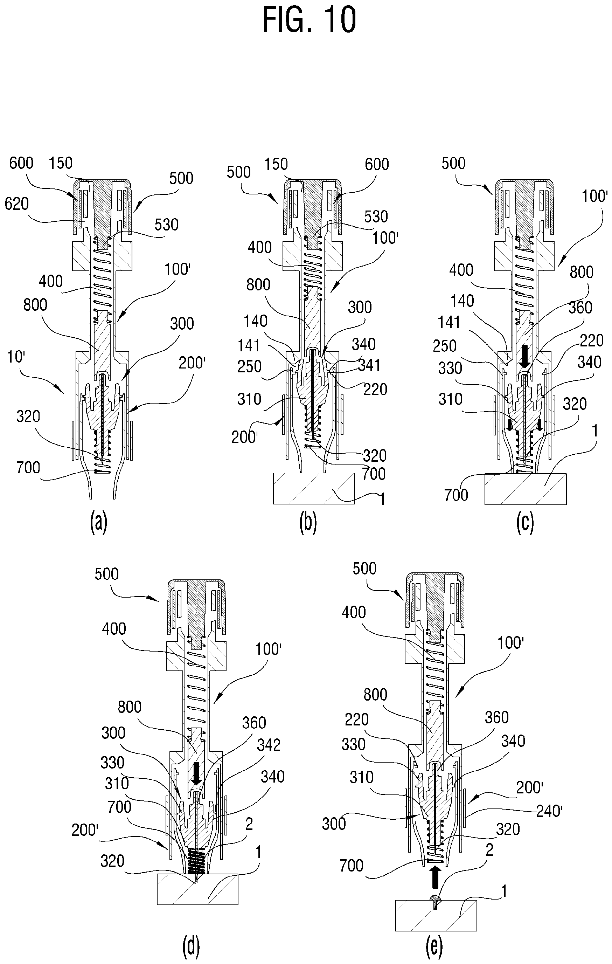

The process of using the blood lancet device 10' in the standby state as shown in FIG. 6 will be described with reference to (a) to (e) of FIG. 10.

(a) of FIG. 10 is a cross-sectional view illustrating the standby state of the blood lancet device 10' in which the needle carrier 300 from which the protective extending portion 350 is removed and the front slider 200' are inserted in the front opening 110' of the barrel 100'. The needle 320 for blood collection is exposed at the front end of the needle carrier 300, and the front slider 200' is prevented from separating frontwards by the functions of the standby lock portion 130' and the standby holding protrusion 210', so that the front slider 200' can move between a standby position and a retraction position in the barrel 100'.

In this state, when the front end of the blood lancet device 10', i.e., the front end of the front slider 200', is placed on the epidermis 1 of a person to be subjected to blood sampling and the barrel 100' is pressurized, the front slider 200' moves in the rearward direction of the barrel 100'.

Meanwhile, a user can rotate the rear end cap 500 to adjust the depth of the needle to be inserted into the epidermis 1 of the person to be subjected to blood sampling. In this case, the user can know the depth of the needle to be inserted, which has been adjusted by the rear end cap 500 through the sign 550 formed on the surface of the rear end cap 500 and the indicator 160 formed on the outer surface of the barrel 100'.

(b) of FIG. 10 illustrates that the blood lancet device 10' moves close to the epidermis 1 of the blood sample and is in a retracted position in which the front slider 200' is moved rearwardly inside the barrel 100'.

In this case, the needle carrier 300 is also moved in the rearward direction by the interaction between the rear end of the front slider 200' and the interlocking holding protrusion 341 formed on the outward projection 340. When the needle carrier 300 moves in the rearward direction of the barrel 100', the elastic transformation portion 330 integrally formed with the needle carrier 300 comes into contact with the trigger portion 140 provided on the inner wall of the barrel 100', and the elastic transformation portion 330 is elastically transformed as being guided to the inner side of the barrel 100' by the slope 141 provided on the trigger portion 140. When the elastic transformation portion 330 is elastically transformed to a certain extent or more, a width between the opposite outward projections 340 becomes narrower than the inner width of the rear end stopper 220 of the front slider 200', thereby releasing a blocking operation of the outward projection 340, and allowing the needle carrier 300 to enter the interior of the front slider 200' and to move in the front end direction.

In addition, as the needle carrier 300 is moved in the rearward direction, the actuation member 800 connected to the interlocking supporting portion 360 is also moved in the rearward direction, the actuation spring 400 interposed between the actuation spring supporter 530 and the actuation member 800 is compressed, and the elastic force of the compressed actuation spring 400 generates a strong force that propels the needle carrier 300 to move down and reach the epidermis 1.

(c) of FIG. 10 illustrates a moment when the needle carrier 300 is fired frontward by the compressed actuation spring 400 and the bumper spring 700 touches the epidermis. The needle carrier 300 receives the elastic force of the actuation spring 400 through the actuation member 800 and moves rectilinearly to the epidermis 1, and the bumper spring 700 provided in the front of the carrier body 310 strikes the epidermis 1. After the bumper spring 700 strikes the epidermis 1, the continued inertial motion of the needle carrier 300 causes the bumper spring 700 to be compressed, and resilience is stored for the retraction of the needle carrier 300.

(d) of FIG. 10 illustrates that the needle 320 for blood collection penetrates into the body of a user and forms the needle hole 2. In this case, the needle 320 for blood collection is fired with minimal transverse shaking from the central axis of the needle carrier 300 by the rectilinear motion of the needle carrier 300 and the actuation member 800 surrounding the interlocking supporting portion 360 of the needle carrier 300, thereby forming the needle hole 2 at an exactly desired point without shaking.

When the inertial force exerted by the needle carrier 300 causes the bumper spring 700 to be compressed to a certain extent or more, the needle 320 for blood collection is propelled rearward by the resilience of the bumper spring 700 and thus pulled out of the epidermis 1, thereby returning to the interior of the front slider 200'.

(e) of FIG. 10 illustrates that the front slider 200' and the needle carrier 300 are returned after the blood collection process. Under the resilience of the compressed the bumper spring 700, the needle 320 for blood collection is quickly pulled out of the epidermis 1 and returns to the interior of the front slider 200', and the front slider 200' is freely positioned between the retraction position and the standby position because it is not under any force in either the upward or downward direction.

In this case, the used needle carrier 300 is located in a front end space of the front slider 200', and the outward projection 340 of the needle carrier 300 is producing friction with the inner surface of the front end of the front slider 200'. Therefore, the needle carrier 300 is not easily separated from the front slider 200' in the front end direction, but may be easily separated by applying a force stronger than the produced friction.

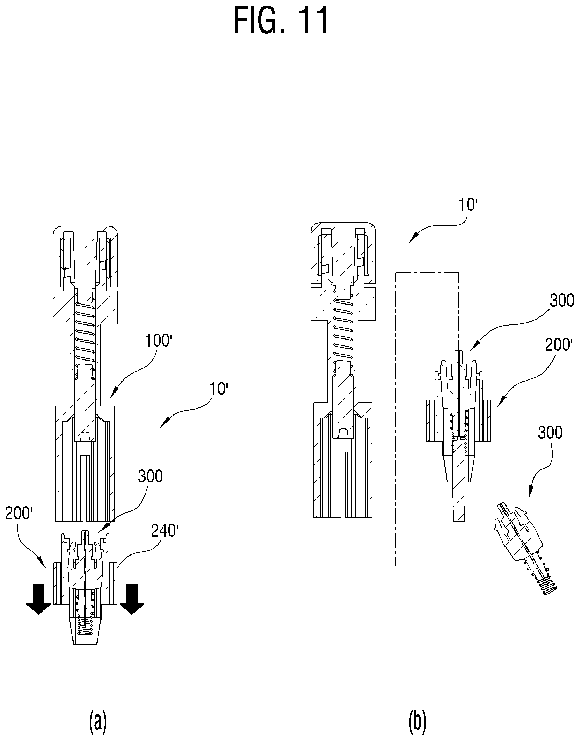

FIGS. 11 (a) and (b) are views illustrating the process of replacing the needle carrier 300 by separating the front slider 200' from the barrel 100' after using the replaceable blood lancet device 10'. Similarly, the process of replacing the needle carrier 300 may also be applied to the replaceable blood lancet device 10 according to the foregoing embodiment shown in FIGS. 1 to 5.

(a) of FIG. 11 illustrates that the front slider 200' of the used blood lancet device 10' is separated from the barrel 100'. When the front slider 200' is in the standby position, a user can pull the withdrawal grip portion 240' in the front end direction, thereby separating the front slider 200' and the needle carrier 300 from the barrel 100'.

(b) of FIG. 11 illustrates the process of inserting a new needle carrier 300 into the separated front slider 200'. The used needle carrier 300 is naturally separated from the interior of the separated front slider 200' by inserting the new needle carrier 300 into the rear end of the front slider 200'. Besides, the replacement of the used needle carrier 300 may also be performed by shaking the blood lancet device 10' to separate the needle carrier 300 out of the blood lancet device 10', or by artificially pressing the rear end of the needle carrier 300. By inserting a new needle carrier 300 into the front slider 200' and then inserting the front slider 200' into the front opening 110' of the barrel 100', the blood lancet device 10' is reusable.

With this process, the replaceable blood lancet device 10' according to the disclosure can be used again by replacing only the needle carrier 300, thereby minimizing resource waste due to consumable parts.

Further, the withdrawal grip portion 240' makes it easy to couple the front slider 200' with the barrel 100', and also makes it easy to remove the front slider 200' from the barrel 100' by gripping the withdrawal grip portion 240' even upon separation.

Further, the blood lancet device 10' includes the actuation spring 400 that elastically pushes the needle carrier 300 in the frontward direction, and the rear end cap 500 that is compressed against the actuation spring 400 to generate an elastic force, so that the blood lancet device 10' can perform blood collection based on the elastic force of the spring.

## Claims

1. A replaceable blood lancet device comprising:
a tubular barrel comprising a front opening;
a needle unit inserted in the front opening of the barrel and slidably movable along an axial line direction; and
an action spring elastically pushing the needle unit in the barrel outwards in a front end direction.

2. The replaceable blood lancet device of claim 1, wherein the barrel comprises a withdrawal opening opened laterally in a predetermined lengthwise section from the front opening to expose the needle unit or cut out in a predetermined section along a longitudinal direction to guide withdrawal of the needle unit.

3. The replaceable blood lancet device of claim 1, wherein the barrel comprises a rear opening, and further comprises a rear end cap provided to open and close the rear opening and being in contact with a rear end of the actuation spring.

4. The replaceable blood lancet device of claim 3, further comprising a needle depth adjuster provided between the barrel and the rear end cap to adjust a position of the rear end cap in an axial line direction.

5. The replaceable blood lancet device of claim 4, wherein the needle depth adjuster comprises a spiral guide portion formed in the barrel, and an engagement moving portion provided in the rear end cap and engaging with the spiral guide portion.

6. The replaceable blood lancet device of claim 3, further comprising:
a stop bushing interposed between the barrel and the rear end cap, rotating with the rear end cap, and comprising a lock resistance portion formed between the stop bushing and the barrel and causing rotation resistance based on engagement therebetween.

7. The replaceable blood lancet device of claim 2, wherein the needle unit comprises:
a tubular front slider inserted in the barrel and movable in the axial line direction, and comprising a front end partially exposed from an end portion of the barrel; and
a needle carrier comprising a carrier body supporting a needle for blood collection to protrude in a front end direction and accommodated in the front slider movably in the axial line direction, a plurality of elastic transformation portions extending rearwards from the carrier body and elastically transformable inwards in a radial direction, and an interlocking holding protrusion protruding from the elastic transformation portion in a direction transverse to the axial line direction, held in a rear end of the front slider, and moving together with the front slider upon rearward movement.

8. The replaceable blood lancet device of claim 7, further comprising:
a stopper protruding inwards in the radial direction of the front slider and kept held in the interlocking holding protrusion; and
a separation preventing protrusion provided in the needle carrier, held in the stopper, and preventing the needle carrier from being separated in a rearward moving direction.

9. The replaceable blood lancet device of claim 7, further comprising a bumper spring mounted to a front of the needle carrier and extending longer than a protruding length of the needle.

10. The replaceable blood lancet device of claim 7, further comprising a standby lock groove provided in one of an inner wall of the barrel and an outer wall of the front slider; and a standby lock portion provided in the other one of the inner wall of the barrel and the outer wall of the front slider, locked to the standby lock groove, and allowing the front slider to be movable within a certain range.

11. The replaceable blood lancet device of claim 7, wherein the front slider further comprises a withdrawal grip portion to grip and withdraw one end of the front slider inserted in the barrel.

12. The replaceable blood lancet device of claim 11, wherein the withdrawal grip portion is connected to the front slider through the withdrawal opening and exposed to the outside of the barrel or annularly surrounding the barrel.

13. The replaceable blood lancet device of claim 7, wherein
the barrel comprises a trigger portion provided therein, and
a slope provided in one of the trigger portion and the elastic transformation portion provided in the needle carrier transforms the elastic transformation portion inwards upon retraction of the needle carrier.

14. A needle unit of a replaceable blood lancet device comprising a tubular barrel, the needle unit comprising:
a tubular front slider inserted in the barrel and movable in the axial line direction, and comprising a front end partially exposed from an end portion of the barrel; and
a needle carrier comprising a carrier body supporting a needle for blood collection to protrude in a front end direction and accommodated in the front slider movably in the axial line direction, a plurality of elastic transformation portions extending rearwards from the carrier body and elastically transformable inwards in a radial direction, and an interlocking holding protrusion protruding from the elastic transformation portion in a direction transverse to the axial line direction, held in a rear end of the front slider, and moving together with the front slider upon rearward movement.

15. The needle unit of claim 14, further comprising:
a stopper protruding inwards in the radial direction of the front slider and kept held in the interlocking holding protrusion; and
a separation preventing protrusion provided in the needle carrier, held in the stopper, and preventing the needle carrier from being separated in a rearward moving direction.

16. The needle unit of claim 14, further comprising a bumper spring mounted to a front of the needle carrier and extending longer than a protruding length of the needle.

17. The needle unit of claim 14, further comprising a standby lock portion provided in an outer wall of the front slider and allowing the front slider to be movable only within a certain range in the barrel.

18. The needle unit of claim 14, wherein the front slider further comprises a withdrawal grip portion to grip and withdraw one end of the front slider inserted in the barrel.

19. The needle unit of claim 18, wherein the withdrawal grip portion is connected to the front slider through the withdrawal opening and exposed to the outside of the barrel or annularly surrounding the barrel.

20. The needle unit of claim 14, wherein the front slider comprises a projection coupling portion to prevent the needle carrier from being separated in a rearward direction.
